Europäisches Patentamt

(19) European Patent Office

Office européen des brevets

(11) Publication number: **0 317 624 B1**

# EUROPEAN PATENT SPECIFICATION

(45) Date of publication of patent specification :
11.12.91 Bulletin 91/50

(51) Int. Cl.[5]: **A61K 37/43, A61K 37/02**

(21) Application number: 88906641.1

(22) Date of filing: 06.06.88

(86) International application number:
PCT/US88/01837

(87) International publication number:
WO 88/09604 15.12.88 Gazette 88/27

(54) THYROTROPIN-RELEASING HORMONE ANALOGS IN CNS INJURY.

(30) Priority: 05.06.87 US 58339

(43) Date of publication of application:
31.05.89 Bulletin 89/22

(45) Publication of the grant of the patent:
11.12.91 Bulletin 91/50

(84) Designated Contracting States:
AT BE CH DE FR GB IT LI LU NL SE

(56) References cited:
EP-A- 188 810
PSYCHONEUROENDOCRIN, vol. 10, 1985;
E.C.GRIFFITHS, pp. 225-235
CIRC. SHOCK, vol. 21, 07 June 1987; T.MCIN-
TOSH et al., p. 376, no. 224

(73) Proprietor : MEDICIS CORPORATION
1747 Pennsylvania Avenue N.W.
Washington, DC 20006 (US)

(72) Inventor : FADEN, Alan, I.
406 Wendy Way
Mill Valley, CA 94941 (US)

(74) Representative : Sternagel, Hans-Günther, Dr.
et al
Patentanwälte Dr. Michael Hann Dr. H.-G.
Sternagel Sander Aue 30
W-5060 Bergisch Gladbach 2 (DE)

## Description

Thyrotropin-releasing hormone (TRH), L-pyroglutamyl-L-histidyl-L-prolineamide, has been found in the spinal cord and has been found to have a variety of effects on the central nervous system. For example, TRH has potent excitatory effects in the spinal cord, thereby increasing neuronal activity and enhancing monosynaptic and polysynaptic reflexes.

TRH improves long-term neurologic outcome following experimental spinal trauma. Consequently, L-pyro-2-aminoadipyl-histidyl-thiazolidine-4-carboxamide and orotyl-L-histidyl-L-prolineamide, synthetic analogs thereof, were studied for such activity in Faden et al., Neurology, Vol. 35, pp. 1331-1334 (1985).
EP-A-0 188 810 discloses the use of several dipeptide derivatives for the production of another pharmaceutical composition for the treatment of possttraumatic nervous system injuries. The only explicitly mentioned preferred compounds described in this document are dioxydihydropyromidylcarbonyl- histidyl-prolineamide (orotyl-histidyl-prolinamide), compound Ia, and the 6-alkyl-5-oxo-thiomorpolin-3-carbonyl-histidyl-prolineamide, compound Ib. There is no disclosure in this document and there is no suggestion that the compounds as presently called for are useful as well for this medical indication.

The object of the present invention is to provide additionnal TRH analogs for the manufacture of a injectable pharmaceutical composition for the treatment of traumatic central nervous system injury suffering from brain or spinal cord trauma. This object is attained by certain TRH analogs to be dealt with later on.

Thus, the present invention provides the use of a thyrotropin-releasing hormone (TRH) analog for the manufacture of an injectable pharmaceutical composition for the treatment of traumatic central nervous system injury suffering from brain or spinal cord trauma, wherein the hormone analog is selected from a 4-(2-oxo-trimethylenimine)-carbonylhistidyl-prolineamide, and a 4-(2-oxo-furan)-carbonyl-histidyl-prolineamide.

The thyrotropin-releasing hormone analog may have a fluorine or iodine substituted histidyl moiety.

Exemplary of such analogs are 2-fluoro and 4-fluoro histidyl TRH analogs. These analogs can be prepared through the fluorination of TRH by conventional techniques.

Iodinated TRH analogs, preferably 2,4-diodo-(Im)-TRH analogs are additionally contemplated in the present invention.

As an effective amount of the thyrotropin-releasing hormone analog of the present invention there is contemplated an amount of analog substantially higher than that required to induce maximal thyrotropin-releasing hormone activity.

Preferably the concentration of the hormone analog in the composition is from 5 mg/cm$^3$ to 10 mg/cm$^3$.

An effective amount of the thyrotropin-releasing hormone analog of the present invention is from 0.2 to 2 mg/kg body weight of the patient administered 2-4 times during the first 48 hours after trauma, 1-2 times daily thereafter. A preferred embodiment of the present invention involves an effective amount of the hormone analog from 0.2 to 1 mg/kg body weight of the patient administered within 24 hours of trauma by 2-4 intravenous or intramuscular injections over 24 hours.

The thyrotropin-releasing hormone analog of the present invention may be administered to the patient in any dosage form convenient under the patient's specific circumstances.

As a parenteral dosage form there is contemplated a dosage unit suitable for intravenous administration which comprises (i) an effective amount of a thyrotropin-releasing hormone analog cited above and (ii) a pharmaceutically acceptable solution.

As a pharmaceutically acceptable solution there is contemplated any solution which is safe for injection and which is biologically inert and hence does not interface with the active ingredient. As such a pharmaceutically acceptable solution may include an isotonic solution suitable for injection into a patient. The isotonic solution may contain water, salt and conventional ingredients such as glucose.

Such a pharmaceutically acceptable solution may contain purified water admixed with preservatives, flavors, colorants, flavor enhancing agents and other excipients. Exemplary of such additives are sodium benzoate, methyl paraben, propylene glycol, glycerin, sorbitol, alcohol, sucrose, saccharin, menthol and citric acid.

4-(2-Oxo-trimethylenimide)-carbonyl-histidyl- prolineamide may be obtained through Dow Chemical Company.

4-(2-Oxo-furan)-carbonyl-histidyl-prolineamide may be obtained through Yamanouchi Pharmaceutical Co., Ltd.

The thyrotropin-releasing hormone analog is administered in a dosage of from 0.2 to 2 mg/kg 2-4 times daily. A more preferred embodiment involves a use, wherein a thyrotropin-releasing hormone analog is administered in a dosage of from 0.2 to 1 mg/kg 2 times daily.

The following illustrate the invention.

## EXAMPLE 1

4-(2-Oxo-trimethylenimine)-carbonyl-histidyl- prolineamide is admixed with 15 cm3 isotonic solution to obtain a final concentration of active ingredient in the solution of 10 mg/cm3.

## EXAMPLE 2

4-(2-Oxo-furan)-carbonyl-histidyl-prolineamide is admixed with 12.5 cm³ isotonic solution to obtain a final concentration of active ingredient in the solution of 7.5 mg/cm³.

## EXAMPLE 3

Induction of tissue protective activity in a patient suffering from traumatic central nervous system injury is accomplished through injection of 15 cm³ of the pharmaceutical preparation of Example 1 4 times daily for 2 days.

## EXAMPLE 4

Induction of tissue protective activity in a patient suffering from traumatic central nervous system injury is accomplished through injection of 10 cm³ of the pharmaceutical preparation of Example 2 2 times daily for 30 days.

## Claims

1. Use of a thyrotropin-releasing hormone (TRH) analog for the manufacture of an injectable pharmaceutical composition for the treatment of traumatic central nervous system injury suffering from brain or spinal cord trauma, wherein the hormone analog is selected from a 4-(2-oxo-trimethylenimine)-carbonyl-histidyl-prolineamide, and a 4-(2-oxo-furan)-carbonyl-histidyl-prolineamide.

2. The use of claim 1, wherein the thyrotropin-releasing hormone analog has a fluorine or iodine substituted histidyl moiety.

3. The use claims 1 to 2, wherein the concentration of the hormone analog in the composition is from 5 mg/cm³ to 10 mg/cm³.

## Patentansprüche

1. Verwendung eines Thyrotropin freisetzenden Hormonanalogen (TRH) zur Herstellung einer injizierbaren pharmazeutischen Zusammensetzung für die Behandlung von traumatischen Schäden des Zentralnervensystems, ausgelöst von Hirn- oder Rückenmarktrauma, wobei das Hormonanaloge aus einem 4-(2-Oxo-trimethylenimin)carbonylhistidylprolineamid und einem 4-(2-Oxo-furan)carbonylhistidylprolieamid ausgewählt ist.

2. Verwendung nach Anspruch 1, **dadurch gekennzeichnet,** daß das Hormonanaloge eine mit Fluor oder Jod substituierte Histidylgruppe aufweist.

3. Verwendung nach Anspruch 1 bis 2, **dadurch gekennzeichnet,** daß die Konzentration des Hormonanalogen in der Zusammensetzung von 5 mg/cm³ bis 10 mg/cm³ beträgt.

## Revendications

1. Utilisation d'un analogue de l'hormone libérant de la thyrotropine HDT pour la préparation d'une composition pharmaceutique injectable pour le traitement de lésions traumatiques du système nerveux central chez un patient souffrant de traumatisme cérébral ou du cordon médullaire, dans lequel l'analogue de l'hormone est sélectionné parmi un 4-(2-oxo-triméthylènimine)-carbonyl-histidyl-prolineamide et un 4-(2-oxo-furanne)-carbonyl-histidyl-prolineamide.

2. Utilisation selon la revendication 1, dans laquelle l'analogue de l'hormone libérant de la thyrotropine présente un groupe histidyle substitué par du fluor ou de l'iode.

3. Utilisation selon les revendications 1 ou 2, dans laquelle la concentration de l'analogue de l'hormone dans la composition est de 5 mg/cm³ à 10 mg/cm³.